# EUROPEAN PATENT SPECIFICATION

(11) **EP 4 387 709 B1**
(45) Date of publication and mention of the grant of the patent: **08.10.2025**
(21) Application number: 22813729.5
(22) Date of filing: 15.11.2022
(51) Int. Cl.: A61N 1/04, A61N 1/36

(54) **ELECTRODE ASSEMBLY WITH NON-HYDROGEL CONDUCTIVE ADHESIVE LAYER**
ELEKTRODENANORDNUNG MIT NICHTHYDROGELLEITENDER HAFTSCHICHT
ENSEMBLE ÉLECTRODE À COUCHE ADHÉSIVE CONDUCTRICE SANS HYDROGEL

(30) Priority: 17.11.2021 US 202163280440 P
(43) Date of publication of application: 26.06.2024
(60) Divisional application: 25199244.2
(73) Proprietor: Novocure GmbH, 6340 Baar (CH)
(72) Inventor: WASSERMAN, Yoram, 319050 Haifa (IL); OBUCHOVSKY, Stas, 31905 Haifa (IL); KUPLENNIK, Nataliya, 31905 Haifa (IL); SHAPIRO, David, 31905 Haifa (IL)
(74) Representative: Boden, Keith McMurray
(86) International application number: PCT/IB2022/061001
(87) International publication number: WO 2023/089484

(56) References cited:
- US-A1- 2010 016 703
- US-A1- 2018 116 546
- US-A1- 2018 117 302
- US-A1- 2019 357 847

## Description

### BACKGROUND

Tumor Treating Fields (TTFields) therapy is a proven approach for treating tumors using alternating electric fields at frequencies between 50 kHz - 1 MHz, more commonly, 100-500 KHz. In current commercial systems, the alternating electric fields are induced by electrode assemblies (e.g., arrays of capacitively coupled electrodes, also called transducer arrays) placed on opposite sides of the subject's body. When an AC voltage is applied between opposing electrode assemblies, an AC current is coupled through the electrode assemblies and into the subject's body. And higher currents are strongly correlated with higher efficacy of treatment.

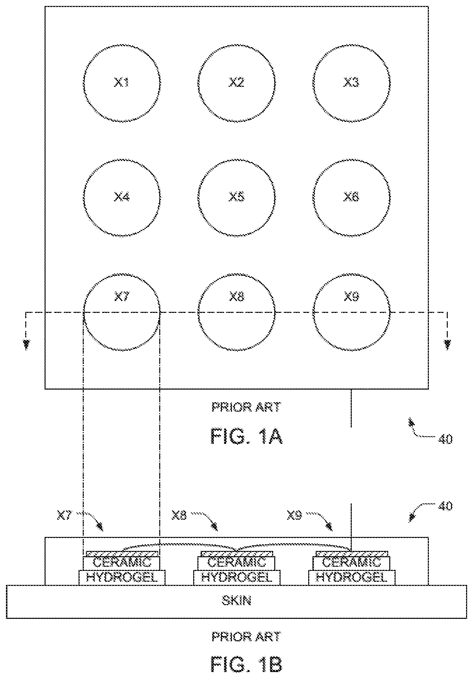

FIG. 1A is a schematic representation of a prior art electrode assembly 40 including nine prior art electrode elements, labeled X1-X9. FIG. 1B is a cross sectional schematic view of electrode elements X7-X9 of the electrode assembly 40, taken along the dashed line in FIG. 1A.

As shown in FIG. 1B, electrode element X7 (taken as exemplary) includes a metal layer (shown with diagonal hatching) and a ceramic (dielectric) layer. A respective layer of electrically conductive hydrogel is provided between each ceramic layer and the subject's skin, to ensure good electrical contact of the electrode elements with the body. An AC voltage from an AC voltage generator (not shown) is applied to the metal layers of electrode elements in opposing electrode assemblies to generate the TTFields in the subject's body. In order to retain the electrode assembly in place during use, an adhesive cover (bandage) is typically provided over the electrode assembly.

During use, the hydrogel and the skin under the electrode elements heat up, and safety considerations require that the skin temperature remain below a safety threshold (e.g., 41°C). Because the vast majority of the heat appears immediately below the electrode elements X1-X9, the prior art electrode assembly has hot spots immediately below the electrode elements, and cooler regions positioned between the electrode elements. And those hot spots limit the amount of current that can be delivered through the prior art electrode assemblies.

The hydrogel layer(s) of the electrode assembly can also present various issues. For example, since the hydrogel has a limited shelf-life, moisture barrier packaging is required, increasing the cost of packaging for the electrode assembly. Additionally, the signal through the hydrogel can vary with the specific moisture content within the hydrogel, and the hydrogel can fail with either too much or too little water. Further, during use, electrode assemblies having hydrogel layers must be changed out frequently, and many patients have adverse reactions (e.g., allergic reactions) to the hydrogel.

US-A-2010/0016703 discloses a bio-electrode for conveying electrical signals to, or from a body which is constructed with two components, a pre-laminated member and a reusable electrode assembly. The pre-laminated member comprises a first substrate layer and a second electrolyte-containing layer. The substrate contacts the body and is constructed of a material that is biocompatible, hydrophilic and inherently electrically semi-conductive or conductive. The electrolyte-containing layer, which does not contact the body, is composed of an adhesive, electrolytic hydrogel.

### SUMMARY

The invention provides an apparatus according to claim 1.

Embodiments of the invention are disclosed in the dependent claims.

### BRIEF DESCRIPTION OF THE DRAWINGS

The foregoing summary, as well as the following description of the disclosure, is better understood when read in conjunction with the appended drawings. For the purpose of illustrating the disclosure, the drawings illustrate some, but not all, alternative embodiments. This disclosure is not limited to the precise arrangements and instrumentalities shown. The following figures, which are incorporated into and constitute part of the specification, assist in explaining the principles of the disclosure.
FIG. 1A is a schematic representation of a prior art electrode assembly.
FIG. 1B is a cross sectional view of electrode elements of the prior art electrode assembly, taken along the dashed line in FIG. 1A.
FIG. 2 is a plan schematic representation of an electrode assembly including electrode elements that is used for applying TTFields to a subject's body.
FIG. 3 is a cross sectional representation of a first embodiment including electrode elements E1, E2, taken along the dashed line in FIG. 2.
FIG. 4A is a thermal image of a prior art electrode assembly.
FIG. 4B is a thermal image of an electrode assembly corresponding to the FIG. 3 embodiment.
FIG. 4C is a graph comparing the thermal properties of the prior art electrode assembly with the FIG. 3 embodiment.
FIG. 5 is a cross sectional representation of a second embodiment including electrode elements E1, E2, taken along the dashed line in FIG. 2.
FIG. 6 is a cross sectional representation of a third embodiment that includes a single electrode element E1.
FIG. 7 is a block diagram of a system incorporating two electrode assemblies that is used for applying TTFields to a subject's body.

### DETAILED DESCRIPTION

### A. Definitions

When the term "about" precedes a numerical value, the numerical value can vary within ±10% unless specified otherwise.

"Anisotropic material" includes any material having a physical property (e.g., a thermal or electrical property) that has a different value when measured in different directions.

"Non-hydrogel" refers to any material that is not a hydrogel, i.e., a material that does not include a crosslinked hydrophilic polymer that is insoluble in water.

"Skin contact layer" means a layer that is configured to contact the skin of a subject (for example, to apply TTFields with the electrode assembly adhered to the skin of the subject).

"Subject" means any living subject, including mammalian subjects such as humans.

The term "particles" has its ordinary meaning, including a very small piece, fragment, or amount of a material. The term "particles" includes but is not limited to carbon flakes, carbon granules, carbon fibers, carbon nanotubes, single-walled carbon nanotubes, multi-walled carbon nanotubes, carbon black powder, graphite powder, carbon nanowires, carbon microcoils.

The present invention can be understood more readily by reference to the following detailed description, examples, drawings, and claims, and their previous and following description. However, it is to be understood that this invention is not limited to the specific apparatuses, devices, systems, and/or methods disclosed unless otherwise specified, and as such, of course, can vary.

Headings are provided for convenience only and are not to be construed to limit the invention in any manner. Embodiments illustrated under any heading or in any portion of the disclosure may be combined with embodiments illustrated under the same or any other heading or other portion of the disclosure.

Any combination of the described elements in all possible variations of those elements is encompassed by the invention unless otherwise indicated or clearly contradicted by context.

### B. Electrode Assembly

This application describes exemplary electrode assemblies that may be used, e.g., for delivering TTFields to a subject's body and treating one or more cancers or tumors located in the subject's body.

When TTFields are applied to a subject's body, the temperature at the subject's body may increase proportionally to the induced electric field. Regulations limit the amount of current that can be driven through a transducer array to an amount that keeps the measured temperature at locations on the subject's body below a temperature threshold. As practiced in the art, the temperature at the location of the transducer arrays on the subject's body is controlled to be below the temperature threshold by reducing the operational current driven by the transducer arrays and reducing the strength of the resulting TTFields. This in turn becomes an over-riding limitation on the TTFields strength that can be used to treat the tumor. Accordingly, there is a need in the art to safely access higher TTField strengths without exceeding the temperature threshold at the subject's skin.

On transducer arrays that comprise multiple electrode elements, the portions of the transducer arrays positioned directly beneath the electrode elements get hotter than the portions of the transducer arrays positioned between the electrode elements. Furthermore, on transducer arrays that comprise multiple electrode elements, higher currents flow through the electrode elements located along the edge of the array compared to the electrode elements located toward the middle of the array. Further still, an electrode element located at a corner or similar sharp bend in the edge of the array will have a higher current than other electrode elements along the edge and near the center of the array. The tendency of a transducer array to drive higher currents through electrode elements located along the edge of the array, and particularly at the comers, is referred to as the "edge effect."

An uneven distribution of current through the transducer array due to either the distribution of the electrode elements or the edge effect can lead to higher temperature zones (or "hot spots"), e.g., at the corners or edges of the transducer array. These hot spots are the locations that reach the threshold temperature first and therefore control the requirement to reduce the current. As such, the generation of hot spots limits the maximum operational current that may be driven by a transducer array, and the strength of the resulting TTFields.

The inventors have now recognized that a need exists for transducer arrays that reduce or minimize uneven distribution of current and allow the application of higher operating currents. Transducer arrays operated with increased current can induce stronger TTFields in the subject's body, ultimately leading to better patient outcomes. The disclosed electrode assemblies allow current and heat to be spread evenly over the array thereby minimizing or eliminating hot spots.

Disclosed embodiments incorporate into the electrode assembly a sheet of material having anisotropic thermal or electrical properties. If the sheet of material has anisotropic thermal properties (for example, a higher thermal conductivity in the plane of the sheet than in the direction perpendicular to the plane of the sheet), then the sheet spreads the heat out more evenly over a larger surface area. If the sheet of material has anisotropic electrical properties (for example, a higher electrical conductivity in the plane of the sheet than in the direction perpendicular to the plane of the sheet), then the sheet spreads the current out more evenly over a larger surface area. In each case, this lowers the temperature of the hot spots and raises the temperature of the cooler regions when a given AC voltage is applied to the electrode assembly (as compared to the prior art configuration described above). Accordingly, the current can be increased (thereby increasing the therapeutic effect) without exceeding the safety temperature threshold at any point on the subject's skin.

In some embodiments, the anisotropic material is anisotropic with respect to electrical conductivity properties. In further embodiments, the anisotropic material is anisotropic with respect to thermal conductivity properties. In certain embodiments, the anisotropic material is anisotropic with respect to both electrical conductivity properties and thermal conductivity properties.

The anisotropic thermal properties include directional thermal properties. Specifically, the sheet of anisotropic material has a first thermal conductivity in a direction that is perpendicular to its front face. And the thermal conductivity of the sheet in directions parallel to the front face is more than two times higher than the first thermal conductivity. In some embodiments, the thermal conductivity in the parallel directions is more than ten times higher than the first thermal conductivity. For example, the thermal conductivity of the sheet in directions that are parallel to the front face may be more than: 1.5 times, 2 times, 3 times, 5 times, 10 times, 20 times, 30 times, 100 times, 200 times, or even more than 1,000 times higher than the first thermal conductivity. In some embodiments, the thermal conductivity of the sheet in directions that are parallel to the front face is between 1.5 times and 1,000 times higher than the first thermal conductivity. In some embodiments, the thermal conductivity of the sheet in directions that are parallel to the front face is between 1.5 times and 20 times higher than the first thermal conductivity. For example, the thermal conductivity of a sheet of pyrolytic graphite in directions that are in the x-y plane is between 10 times and 20 times higher than its thermal conductivity in the perpendicular z-direction.

The anisotropic electrical properties include directional electrical properties. Specifically, the sheet has a first resistance in a direction that is perpendicular to its front face. And resistance of the sheet in directions parallel to the front face is less than the first resistance. In some exemplary embodiments, the resistance in the parallel directions is less than half of the first resistance or less than 10% of the first resistance. The resistance of the sheet in directions that are parallel to the front face may be less than: 75%, 50%, 40%, 30%, 20%, 10%, 5%, 1%, 0.5%, 0.1%, or even less than 0.05% of the first resistance. In some embodiments, the resistance of the sheet in directions that are parallel to the front face is between 0.05% and 75% of the first resistance. In some embodiments, the resistance of the sheet in directions that are parallel to the front face is between 0.05% and 10 % of the first resistance. For example, the electrical resistivity of a sheet of pyrolytic graphite in directions that are in the x-y plane is approximately three orders of magnitude (1,000 times) lower than its electrical resistivity in the perpendicular z-direction.

In some embodiments (e.g., when the sheet of anisotropic material is a sheet of pyrolytic graphite), the sheet of anisotropic material has both anisotropic electrical properties and anisotropic thermal properties.

In some embodiments (e.g., when the sheet of anisotropic material is a sheet of pyrolytic graphite), the sheet of anisotropic material is nonmetallic. These embodiments are particularly advantageous in situations where preventing the transfer of ions into a subject's body is desirable. More specifically, using a metallic sheet could result in the transfer of metal ions into a subject's body. In situations where this is not desirable, embodiments that use nonmetallic sheets of anisotropic material are preferable.

In addition to the anisotropic material, embodiments of the disclosed electrode assemblies feature a layer of non-hydrogel conductive adhesive positioned between the skin-facing surface of an electrode element and the outwardly facing surface of the layer of anisotropic material. This upper non-hydrogel conductive adhesive improves conductivity along the z-axis direction, i.e., electrical conductivity is enhanced in a direction that is perpendicular to a plane of the layer of anisotropic material.

In general, but without being bound by any theory, the anisotropic material enables a voltage difference from the center of the surface to the edges, allowing for current flow to spread more evenly. This decreases the effect of shielding (edge effect) and decreases the presence of hot spots in the skin contact layer. However, if current is driven along a small area, contact resistance between the electrode and the upper adhesive layer generates heat at that area. Although the contact area can be increased such that the heating generated by the contact resistance is not a limiting factor, a larger area also increases the amount of shielding generated by the applied voltage. This, as previously discussed, leads to uneven temperature along the skin contact area.

The inventors discovered that heat generated by the contact resistance of the electrode and the upper adhesive can be reduced by decreasing the z-direction resistance of the upper adhesive layer. This can be achieved by introducing a higher dielectric material into the upper adhesive layer of by incorporating materials with increased z-direction conductivity. Addition of a dielectric material into the upper adhesive layer introduces a capacitive component. As stronger electric fields are generated, the resistivity of the capacitor decreases and approaches zero, increasing the conductivity of the material while minimizing the heat generated at the electrode-upper adhesive contact. z-direction conductivity can also be enhanced by increasing the surface area of the conductive component within the adhesive in the z-direction. Structures such as carbon nanowires, for example, tend to lie flat in the plane and have excellent x-y conductivity along the length of the wire. To some extent, they may also enhance z-direction conductivity at points of contact between the wires. Carbon nanotubes may function similarly. It is contemplated that other shapes with 3D structures and significant 3D footprint (in terms of filling 3D volume) such as carbon microcoils may exhibit improved conductivity in the z-direction. Such materials are referred to herein as conductive materials having 3D carbon structures.

Accordingly, it is contemplated that embodiments that include the sheet of anisotropic material may aid in avoiding or reducing overheating of the electrodes and associated discomfort on the skin by dissipating both electrical current and heat in a lateral (in-plane) direction rather than passing directly through the layer (in a direction perpendicular to the plane of the skin contact layer) in a concentrated manner. And, it is further contemplated that decreasing the z-direction resistance of the upper adhesive layer can reduce the heat generated by the contact resistance of the electrode and the upper adhesive layer and further enable the realization of the beneficial effect of delivering higher operating currents for any chosen temperature (e.g. staying below the threshold temperature, ~41 °C, or choosing to run at a maximum skin surface temperature of from 38-41 °C, or from about 38-40 °C), especially if it is desired to replace a hydrogel with a non-hydrogel conductive adhesive in that layer.

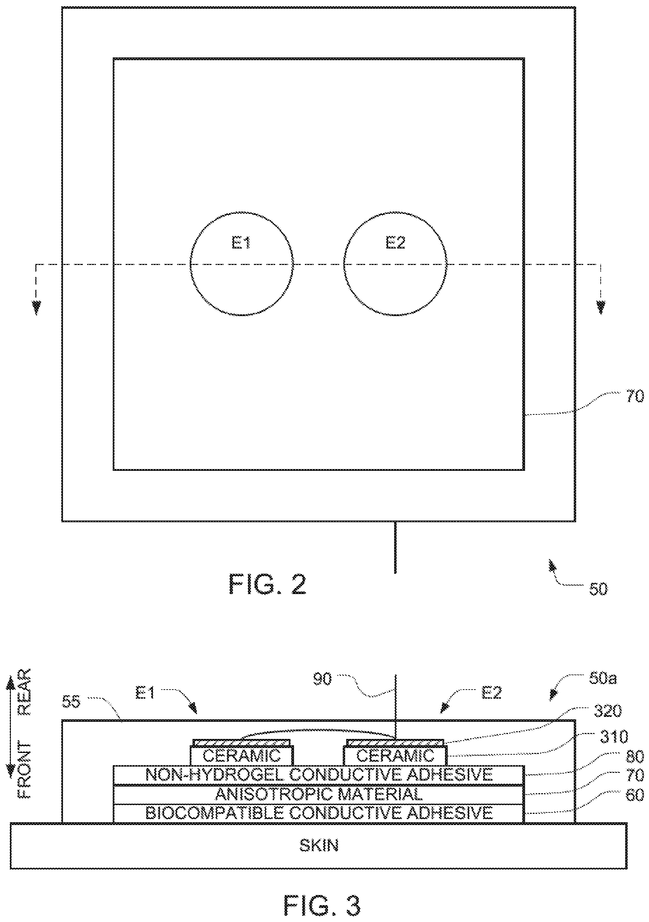

FIG. 2 is a schematic representation of an electrode assembly 50 of an embodiment including electrode elements used for applying TTFields to a subject's body. In FIG. 2, only two electrode elements labeled E1 and E2 are shown, but additional electrode elements may be included in the electrode assembly 50. In alternative embodiments, the electrode assembly 50 includes only a single electrode element. Notably, FIG. 2 depicts an electrode assembly 50 generically, and those electrode assemblies E1 and E2 can have different configurations (e.g., as described below in connection with FIGs. 3-7).

FIG. 3 is a cross sectional representation of a first embodiment of an electrode assembly 50a including electrode elements E1, E2, taken along the dashed line in FIG. 2. In the FIG. 3 embodiment, the electrode assembly 50a includes a sheet of anisotropic material 70 having a front face (facing towards the subject's skin in FIG. 3) and a rear face. This sheet 70 has a first thermal conductivity in a direction that is perpendicular to the front face. Thermal conductivity of the sheet 70 in directions that are parallel to the front face can be more than two times higher than the first thermal conductivity. In some embodiments, the thermal conductivity of the sheet 70 in directions that are parallel to the front face is more than ten times higher than the first thermal conductivity. The sheet 70 in the FIG. 3 embodiment is also anisotropic in another respect. More specifically, the sheet 70 has a first resistance in a direction that is perpendicular to the front face, and the resistance of the sheet in directions that are parallel to the front face can be less than half of the first resistance. In some embodiments, the resistance of the sheet in directions that are parallel to the front face is less than 10% of the first resistance.

In some embodiments, the sheet of anisotropic material 70 is a sheet of pyrolytic graphite (such as, for example, Pyrolytic Graphite Sheet (PGS), available from Panasonic Industry, Kadoma, Osaka, Japan). Thermal conductivity of pyrolytic graphite sheets in directions that are parallel to the front face of those sheets (i.e., in the x-y plane) is typically more than 50 times higher than the thermal conductivity of those sheets in directions that are perpendicular to the front face (i.e., in the z direction). Electrical resistivity of pyrolytic graphite sheets in directions that are parallel to the front face of those sheets (i.e., in the x-y plane) is typically less than 2% of the electrical resistivity of those sheets in directions that are perpendicular to the front face (i.e., in the z direction).

In other embodiments, the sheet of anisotropic material 70 is graphite foil made from compressed high purity exfoliated mineral graphite (e.g., MinGraph^{®} 2010A Flexible Graphite, available from Mineral Seal Corp., Tucson, Arizona, USA), or graphitized polymer film, e.g., graphitized polyimide film, (including, but not limited to, that supplied by Kaneka Corp., Moka, Tochigi, Japan). In other embodiments, the anisotropic material may be pyrolytic carbon. In other embodiments, the anisotropic material may be boron nitride. Other embodiments may utilize sheets of other conducting materials with anisotropic properties. In some embodiments (e.g., when the sheet of anisotropic material is a sheet of a synthetic graphite, such as pyrolytic graphite or the compressed high purity exfoliated mineral graphite), the sheet of anisotropic material 70 is nonmetallic.

The electrode assembly 50a further includes a front layer of biocompatible conductive adhesive material 60 disposed on the front face of the sheet 70. The front layer of material 60 can be configured to ensure good electrical contact between the device and the body. In some embodiments, the front layer of material 60 should cover the entire front face of the sheet of anisotropic material 70. The front layer of material 60 may be the same size or larger (i.e. cover the same area or more) than the sheet of anisotropic material 70. In some embodiments, the front layer of conductive material 60 does not comprise water. In certain embodiments, the front layer of conductive material 60 is a non-hydrogel conductive material. In other embodiments, the front layer of conductive material 60 comprises a hydrogel. In these embodiments, the hydrogel may have a thickness between about 20 and 2000 µm, such as, from 100 to 1000 µm, or even 300 to 500 µm. In some embodiments, the front layer of conductive material 60 is a non-hydrogel biocompatible conductive adhesive. In some embodiments, the front layer of conductive material 60 is a non-hydrogel biocompatible conductive adhesive such as the developmental product FLX068983 - FLEXcon^{®} OMNI-WAVE^{™} TT 200 BLACK H-502 150 POLY H-9 44PP-8 from FLEXcon, Spencer, MA, USA, or other such OMNI-WAVE products from FLEXcon; or AReare^{®} 8006 electrically conductive adhesive composition manufactured and sold by Adhesives Research, Inc. (Glen Rock, PA, USA). Non-hydrogel conductive adhesives may comprise a waterless polymer with adhesive properties and carbon particles, powder, fibers, flakes, nanotubes, nanowires or microcoils. The adhesive polymer may be, for example, an acrylic polymer or a silicone polymer, or combination thereof, which may be available as acrylic- or silicone-based carbon-filled adhesive tapes. The adhesive may additionally include one or more conductive polymer (for example, polyaniline (PANI), or poly(3,4-ethylenedioxythiophene (PEDOT), or others known in the art). Preferably, the conductive filler in the front layer of conductive material 60 is non-metallic. In these embodiments, the biocompatible conductive adhesive may have a thickness between 10 and 2,000 µm, such as, from 20 to 1000 µm, or even 30 to 400 µm.

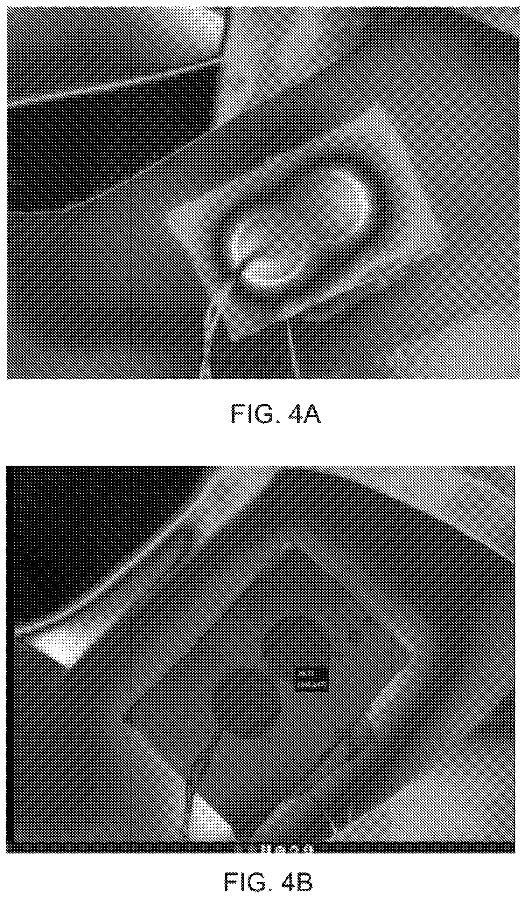

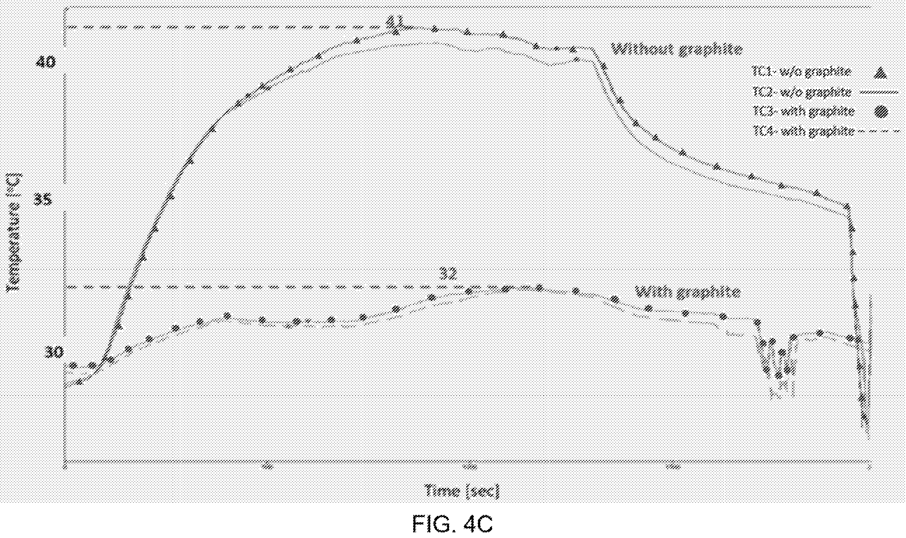

In the FIG. 3 embodiments (or embodiments shown in FIGs. 4-7), the front or skin contact layer of the electrode assembly does not comprise a latex rubber polymer. In some embodiments, the front or skin contact layer does not comprise silver or silver chloride. In additional embodiments, the front or skin contact layer is releasably connected to the layer of anisotropic material. In these embodiments, the skin contact layer can be selectively detached from the anisotropic material and replaced with a new skin contact layer (for example, when a maximum/threshold duration of use is approached or met).

In certain embodiments, the front or skin contact layer can comprise a biocompatible conductive adhesive having a thickness ranging from about 20 µm to about 2,000 µm, e.g., about 30 µm to about 2000 µm, such as from about 30 µm to about 70 µm, or from about 45 µm to about 55 µm.

Optionally, in exemplary embodiments, the apparatus can further comprise a release liner that covers the skin contact layer. In these embodiments, it is contemplated that, prior to use, the apparatus can be provided with the release liner to ensure that the skin contact layer does not adhere to undesirable surfaces or locations. Immediately prior to use, the release liner can be removed, and the skin contact layer can be positioned in contact with the skin of the patient.

The electrode assembly 50a further includes a first electrode element E1 positioned behind the sheet 70. The first electrode element E1 has a first front face disposed in electrical contact with the rear face of the sheet 70. In the FIG. 3 embodiment, the first electrode element E1 includes a first layer of dielectric (e.g., ceramic) material 310 having a front face and a rear face, and a first layer of metal 320 disposed on the rear face of the first layer of dielectric material 310. The front face of the first layer of dielectric material 310 is the first front face of the first electrode element E1.

The dielectric material need not be ceramic. In some aspects, for example, the dielectric material 310 can comprise polymer (e.g., high dielectric constant polymer). Accordingly, it should be understood that, in all embodiments disclosed herein, the dielectric material 310 referred to and shown in the drawings as ceramic can be any suitable dielectric material (for example, a polymer layer having a dielectric constant of at least 10, or another material having a dielectric constant of at least 10).

In some embodiments, the layer of dielectric material 310 can have a dielectric constant ranging from 10 to 50,000. In some embodiments, the layer of dielectric material 310 comprises a high dielectric polymer material such as poly(vinylidene fluoride-trifluoroethylene-chlorotrifluoroethylene) and/or poly(vinylidene fluoride-trifluoroethylene-1-chlorofluoroethylene). Those two polymers are abbreviated herein as "Poly(VDF-TrFE-CTFE)" and "Poly(VDF-TrFE-CFE)," respectively. These embodiments are particularly advantageous because the dielectric constant of these materials is on the order of 40. In some embodiments, the polymer layer can be poly(vinylidene fluoride-trifluoroethylene-chlorotrifluoroethylene-chlorofluoroethylene) or "Poly(VDF-TrFE-CTFE-CFE)."

In some embodiments, the layer of dielectric material 310 comprises a terpolymer comprising polymerized units of monomers such as VDF, TrFE, CFE and/or CTFE in any suitable molar ratio. Suitable terpolymers include those, for example, having 30 to 80 mol% VDF, 5 to 60 mol% TrFE, with CFE and/or CTFE constituting the balance of the mol% of the terpolymer.

In some embodiments, the sheet 70 has a centroid, and the centroid of the first front face of the first electrode element E1 is positioned less than 3 cm away from the centroid of the sheet 70. In some embodiments, the sheet 70 has a centroid and a dimension parallel to the rear face of the sheet 70 (e.g., a length or a width), and the centroid of the first front face of the first electrode element E1 is positioned away from the centroid of the sheet 70 by less than 30%, or by less than 10% of the dimension.

The electrode assembly 50a further includes a first layer of non-hydrogel conductive adhesive 80 positioned between the first front face of the first electrode element E1 (i.e., the front face of the first layer of dielectric material 310) and the rear face of the sheet 70. The first layer of non-hydrogel conductive adhesive 80 facilitates the electrical contact between the first front face of the first electrode element E1 and the rear face of the sheet 70. In the illustrated embodiment, the layer of conductive material 80 is a layer of non-hydrogel conductive adhesive. In some embodiments, a different conductive material (e.g., conductive grease, conductive adhesives, conductive tape, etc.) could be used. In some embodiments, the layer of non-hydrogel conductive adhesive 80 may be the developmental product FLX068983 - FLEXcon^{®} OMNI-WAVE^{™} TT 200 BLACK H-502 150 POLY H-9 44PP-8 from FLEXcon, discussed above, or other such OMNI-WAVE products from FLEXcon; or, alternatively, ARcare^{®} 8006 electrically conductive adhesive composition manufactured and sold by Adhesives Research, Inc. (Glen Rock, PA, USA). Non-hydrogel conductive adhesives may comprise a waterless polymer with adhesive properties. The adhesive polymer may be, for example, an acrylic polymer or a silicone polymer, or combination thereof. In these embodiments, the first layer of conductive adhesive may have a thickness between 10 and 2,000 µm, such as, from 20 to 1000 µm, or even 30 to 400 µm.

In some embodiments, the electrode assembly 50a or other described electrode assemblies feature a first layer of non-hydrogel conductive adhesive comprising a material that facilitates electrical conductivity in a direction that is perpendicular to a plane of the layer of anisotropic material (the z-direction). Non-hydrogel conductive adhesives may comprise a waterless polymer with adhesive properties and carbon particles, powder, fibers, flakes, nanotubes, or nanowires, or combination thereof. In some embodiments, the non-hydrogel conductive adhesive includes conductive particles having shapes with 3D structures and significant 3D footprint (in terms of filling 3D volume) for enhancing conductivity in the z-direction, such as, for example, carbon microcoils. The adhesive polymer may be, for example, an acrylic polymer or a silicone polymer, or combination thereof, which may be available as acrylic- or silicone-based carbon-filled adhesive tapes. The adhesive may additionally include one or more conductive polymer (for example, polyaniline (PANI), or poly(3,4-ethylenedioxythiophene (PEDOT), or others known in the art). The conductive filler in the first layer of conductive material 80 may also be non-metallic.

The electrode assembly 50a may optionally include one or more additional electrode elements. In the illustrated embodiment, the electrode assembly 50a includes a second electrode element E2 positioned behind the sheet 70. The second electrode element E2 has a second front face disposed in electrical contact with the rear face of the sheet 70. The two electrode elements E1, E2 in FIG. 3 have identical structures. Thus, the second electrode element E2 includes a second layer of dielectric (e.g., ceramic) material 310 having a front face and a rear face, and a second layer of metal 320 disposed on the rear face of the second layer of dielectric material 310. The front face of the second layer of dielectric material 310 is the second front face of the second electrode element E2.

The first layer of non-hydrogel conductive adhesive 80 is positioned between the second front face of the second electrode element E2 (i.e., the front face of the second layer of dielectric material 310) and the rear face of the sheet 70. The first layer of non-hydrogel conductive adhesive 80 facilitates the electrical contact between the second front face of the second electrode element E2 and the rear face of the sheet 70. As described for E1, and as shown in FIG. 3, the conductive material 80 may be a layer of a non-hydrogel material, e.g., a material that in some embodiments does not comprise water. In some embodiments, a different conductive material may be used (e.g., conductive grease, conductive adhesive including the non-hydrogel conductive adhesives described above, conductive tape, conductive composite, etc.).

The metal layers 320 of all of the electrode elements (i.e., E1 and E2 in the illustrated embodiment), may be wired together (e.g., using wires, traces on a flex circuit, etc.) to a lead 90. The lead 90 supplies an AC voltage from an AC voltage generator (not shown) to the electrode elements to generate the TTFields when the electrode assembly 50a is affixed to the subject's body for treatment.

Optionally, the electrode assembly 50a includes a flexible self-adhesive backing 55 configured to support the sheet 70, the first electrode element E1 (and any other electrode elements present in the electrode assembly), and the front layer of biocompatible conductive material 60 so that the front layer of biocompatible conductive material 60 can be positioned against the subject's skin.

In some embodiments, by using a biocompatible conductive layer as a skin contact layer, it is contemplated that additional backing and/or cover layers (such as, for example self-adhesive backing 55) can be omitted (with respect to any embodiments shown in FIGs. 3-7). In these embodiments, the biocompatible conductive layer can provide sufficient adhesion to the skin such that it is unnecessary to provide additional layers to maintain a desired position of the electrode assembly on the body of the subject, thereby improving ease of use and decreasing the overall cost of manufacture and use.

The superior performance of the FIG. 3 embodiment is demonstrated in FIGs. 4A, 4B, and 4C. FIG. 4A is a thermal image of a prior art electrode assembly that includes two electrode elements and a layer of hydrogel disposed on the front faces of the electrode elements (see, e.g., FIG. 1B). There is no sheet of anisotropic material between the front faces of the electrode elements and the rear face of the layer of hydrogel. In use, the front face of the layer of hydrogel is positioned on the subject's skin. FIG. 4A shows hot spots generated in the areas that correspond to the electrode elements.

FIG. 4B is a thermal image of an electrode assembly corresponding to the FIG. 3 embodiment where pyrolytic graphite was used as the anisotropic material. FIG. 4B shows that hot spots such as those generated in the prior art electrode assembly have been minimized, and also that the maximum temperature has been reduced. FIG. 4C is a graph comparing the thermal performance of the FIG. 3 embodiment (utilizing pyrolytic graphite as the anisotropic material) with the prior art (no anisotropic material) for the same applied current (500 mA). Notably, the hottest portion of the prior art electrode assembly was 41°C. But when the same 500 mA current was applied to the FIG. 3 embodiment, the hottest portion of the electrode assembly was only 32°C. Similar experiments were performed utilizing graphite foil made from compressed high purity exfoliated mineral graphite as the anisotropic material, with similar results.

In a related experiment, optimized conventional arrays (no anisotropic material), running with 2 A applied current, ran up to the maximum 40°C average temperature, and were thereby limited. The same type of array with an added pyrolytic graphite sheet (in the manner of the FIG. 3 embodiment) was able to run at an increased power level (with 3 A applied current), and ran at 38°C average temperature, 2-3°C below the temperature threshold limit. This result suggests that the inventive apparatus and methods described herein should be able to achieve more beneficial treatment results by operating at higher applied currents, while maintaining temperature at the skin surface to be below the threshold temperature (about 41°C).

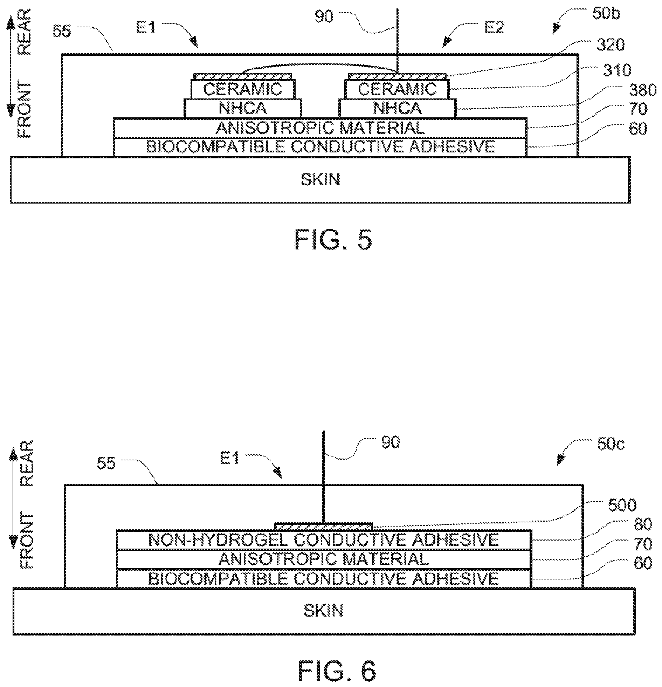

FIG. 5 is a cross sectional representation of a second embodiment of an electrode assembly 50b including electrode elements E1, E2, taken along the dashed line in FIG. 2. The FIG. 5 embodiment is similar to the FIG. 3 embodiment in all respects except as follows. The FIG. 3 embodiment includes a large continuous layer of non-hydrogel conductive adhesive material 80 positioned between the sheet 70 and the front faces of both the first and second electrode elements E1 and E2. In contrast, the FIG. 5 embodiment includes a separate region of non-hydrogel conductive adhesive material 380 (abbreviated "NHCA") for each individual electrode element. Thus, the FIG. 5 embodiment includes a first layer of non-hydrogel conductive adhesive material 380 positioned between the first front face of the first electrode element E1 and the rear face of the sheet 70, and also includes a second layer of non-hydrogel conductive adhesive material 380 positioned between the second front face of the second electrode element E2 and the rear face of the sheet 70. The first and second layers of non-hydrogel conductive adhesive material 380 facilitate the electrical contact between the respective electrode front faces and the rear face of the sheet 70. In the embodiment illustrated in FIG. 5, the layers of non-hydrogel conductive adhesive material 380 are layers of material that in some embodiments do not comprise a hydrogel and in some aspects do not comprise water. In additional embodiments, non-hydrogel conductive adhesive material 70 can include conductive particles such as 3D carbon structures for enhancing conductivity in the z-direction as described above and elsewhere herein. In some embodiments, different conductive materials (e.g., conductive grease, conductive adhesive including the non-hydrogel conductive adhesives described above, conductive tape, conductive composite, etc.) could be used.

As in the FIG. 3 embodiment, the current in the FIG. 5 embodiment is still concentrated in the upper layers of non-hydrogel conductive adhesive material 380 only in the areas below the electrode elements. The sheet of anisotropic material 70 spreads out the heat and the current as described above in connection with the FIG. 3 embodiment, which eliminates or at least minimizes hot spots. This means that for a given applied AC voltage, the hottest point beneath the electrode assembly in the FIG. 5 embodiment will be at a lower temperature than the hottest point beneath the electrode assembly in the FIG. 1 prior art embodiment. Accordingly, the current can be increased (with respect to the prior art current) without exceeding the safety temperature threshold at any point beneath the electrode assembly in the FIG. 5 embodiment. This increase in current will advantageously increase the efficacy of the TTFields treatment.

FIG. 6 is a cross sectional representation of a third embodiment of an electrode assembly 50c that includes a single electrode element E1. The embodiment of FIG. 6 is similar to the embodiment of FIG. 3, except the FIG. 6 embodiment does not include the layer of dielectric material. In the FIG. 6 embodiment, the electrode assembly 50c includes a sheet of anisotropic material 70 having a front face (facing towards the subject's skin in FIG. 6) and a rear face. This sheet 70 is similar to the sheet 70 described above in connection with FIG. 3. In some embodiments, the sheet of anisotropic material 70 is a sheet of synthetic graphite. In some embodiments, the sheet of anisotropic material 70 is a sheet of pyrolytic graphite. In other embodiments, the sheet of anisotropic material 70 is a sheet of pyrolytic carbon. In other embodiments, the sheet of anisotropic material 70 is graphite foil made from compressed high purity exfoliated mineral graphite (e.g., MinGraph^{®} 2010A Flexible Graphite). In other embodiments, the anisotropic material may be boron nitride. In other embodiments, the sheet of anisotropic material 70 is a sheet of another conductive anisotropic material.

The electrode assembly 50c further includes a front layer of biocompatible conductive material 60 disposed on the front face of the sheet 70. The front layer of conductive material 60 is configured to ensure good electrical contact between the device and the body. In one embodiment, the front layer of conductive material 60 should cover the entire front face of the sheet of anisotropic material 70. The front layer of conductive material 60 may be the same size or larger (i.e., cover the same area or larger) than the sheet of anisotropic material 70. In some embodiments, the front layer of conductive material comprises a non-hydrogel conductive material, including for example conductive materials that do not comprise water. In other embodiments, the front layer of conductive material 60 comprises hydrogel. In these embodiments, the hydrogel may have a thickness between 20 and 2000 µm, such as, from 50 to 1000 µm, or even 100 to 500 µm. In some embodiments, the front layer of conductive material 60 is a non-hydrogel biocompatible conductive adhesive, such as those described above, and including the OMNI-WAVE^{™} products from FLEXcon, or the ARcare^{®} products from Adhesives Research, Inc., discussed above. Non-hydrogel conductive adhesives may comprise a waterless polymer with adhesive properties (for example, an acrylic polymer or a silicone polymer, or combination thereof) and a conductive filler. The conductive filler in the front layer of conductive material 60 should be non-metallic. In these embodiments, the biocompatible conductive adhesive may have a thickness between 10 and 2,000 µm, such as, from 20 to 1000 µm, or even 30 to 400 µm.

The electrode assembly 50c further includes a first electrode element E1 positioned behind the sheet 70. The first electrode element E1 includes a piece of metal 500 having a front face disposed in electrical contact with the rear face of the sheet 70.. In the FIG. 6 embodiment, the front face of the piece of metal 500 is the first front face of the first electrode element E1. Accordingly, the FIG. 6 embodiment differs from the FIG. 3A or FIG. 5 embodiments by omitting a layer of dielectric material. The positional relationship between the first electrode element E1 and the sheet 70 in this FIG. 6 embodiment may be as described above in connection with FIG. 3.

The electrode assembly 50c further includes a first layer of non-hydrogel conductive adhesive material 80 positioned between the first front face of the first electrode element E1 (i.e., the front face of the piece of metal 500) and the rear face of the sheet 70. The first layer of non-hydrogel conductive adhesive material 80 facilitates the electrical contact between the first front face of the first electrode element E1 and the rear face of the sheet 70. In the illustrated embodiment, the layer of non-hydrogel conductive adhesive material 80 is a layer of material that in some embodiments does not comprise a hydrogel and in some aspects does not comprise water. In additional embodiments, non-hydrogel conductive adhesive material 80 can include conductive particles such as 3D carbon structures for enhancing conductivity in the z-direction as described above. In some embodiments, different conductive materials (e.g., conductive grease, conductive adhesive including the non-hydrogel conductive adhesives described above, conductive tape, conductive composite, etc.) could be used.

The piece of metal 500 of the electrode element E1 is wired (e.g., using wires, traces on a flex circuit, etc.) to a lead 90, which supplies an AC voltage from an AC voltage generator (not shown) to the electrode element to generate the TTFields when the electrode assembly 50c is affixed to the subject's body for treatment. The electrode assembly 50c may optionally include one or more additional electrode elements having a structure identical to electrode element E1 and positioned to have the same functionality. In such case, the pieces of metal 500 of all the electrode elements may be wired together (e.g., using wires, traces on a flex circuit, etc.) to the lead 90.

In some embodiments that include only a single electrode element E1, the area of the sheet 70 is larger (e.g., at least 2, or at least 4, or at least 10 times larger) than the area of the electrode element E1. In some embodiments that include a plurality of electrode elements (not shown) the area of the sheet 70 is larger (e.g., at least 2, 4, or 10 times larger) than the collective area of all of the electrode elements. When an AC voltage is applied to the electrode elements, the heat spreads out across the entire sheet 70, which minimizes or eliminates hot spots.

Similar to the FIG. 3 embodiment, the sheet of anisotropic material 70 in the FIG. 6 embodiment spreads out the heat and the current as described above in connection with the FIG. 3 embodiment, which eliminates or at least minimizes hot spots. This means that for a given applied AC voltage, the hottest point beneath the electrode assembly in the FIG. 6 embodiment will have a lower temperature than the hottest point beneath the electrode assembly in the FIG. 1 prior art embodiment. Accordingly, the current can be increased (with respect to the prior art current) without exceeding the safety temperature threshold at any point beneath the electrode assembly in the FIG. 6 embodiment. This increase in current will advantageously increase the efficacy of the TTFields treatment.

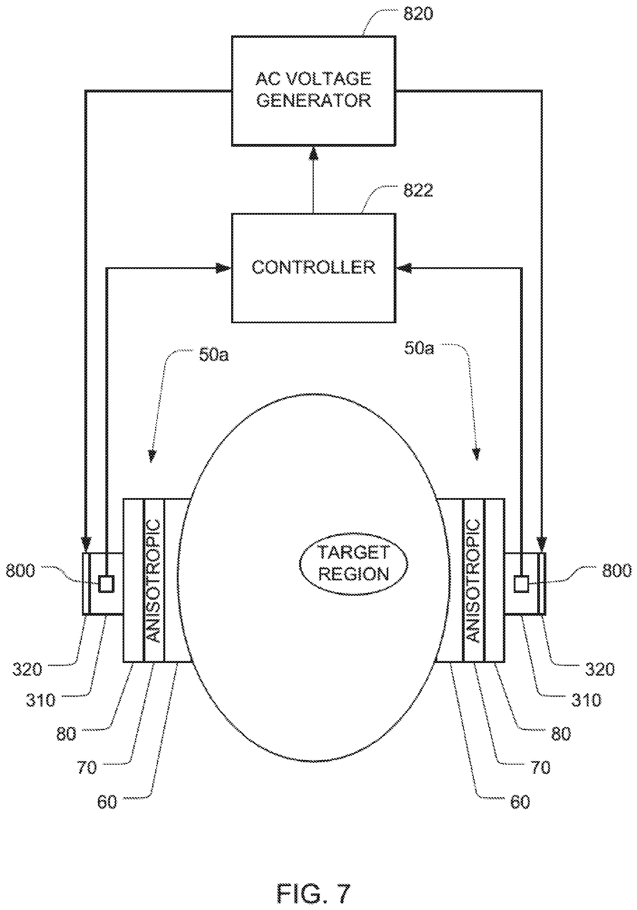

FIG. 7 shows how a pair of the FIG. 3 electrode assemblies 50a may be used to apply an alternating electric field to a target region in the subject's body. (Note that any of the electrode assemblies described above in connection with FIGs. 3-6 may be used instead of the FIG. 3 electrode assemblies 50a shown here). The method includes positioning a first electrode assembly 50a at a first position on or in the subject's body. (In the example depicted in FIG. 7, the first electrode assembly 50a is positioned on the subject's skin at the right of the subject's head facing a target region, e.g., a tumor.) The first electrode assembly 50a may be constructed as described earlier. In the FIG. 7 embodiment, the first electrode assembly 50a includes a first sheet of anisotropic material 70 having a first front face and a first rear face. The first sheet 70 has a first thermal conductivity in a direction that is perpendicular to the first front face. Thermal conductivity of the first sheet 70 in directions that are parallel to the first front face of the sheet 70 is more than two times higher than the first thermal conductivity. The first sheet 70 has a first resistance in a direction that is perpendicular to the front face, and the resistance of the first sheet in directions that are parallel to the front face is less than half of the first resistance. During use, the first electrode assembly 50a is positioned so that the first front face of the first sheet 70 faces the target region.

The method also includes positioning a second electrode assembly 50a at a second position in or on the subject's body. (In the example depicted in FIG. 7, the second electrode assembly 50a is positioned on the subject's skin at the left of the subject's head facing the target region.) The second electrode assembly 50a may be constructed as described earlier herein. In the FIG. 7 embodiment, the second electrode assembly 50a includes a second sheet 70 of anisotropic material 70 having a second front face and a second rear face. The second sheet 70 has a second thermal conductivity in a direction that is perpendicular to the second front face. Thermal conductivity of the second sheet 70 in directions that are parallel to the second front face of the sheet 70 is more than two times higher than the second thermal conductivity. The second sheet 70 has a second resistance in a direction that is perpendicular to the front face, and the resistance of the second sheet in directions that are parallel to the front face is less than half of the second resistance. During use, the second electrode assembly 50a is positioned so that the second front face of the second sheet 70 faces the target region.

The method further includes applying an alternating voltage between the first electrode assembly 50a and the second electrode assembly 50a. The applying is performed after positioning the first electrode assembly 50a and the second electrode assembly 50a. The applying may be implemented by applying the alternating voltage between (i) a first electrode element disposed in electrical contact with the first rear face of the first sheet 70 and (ii) a second electrode element disposed in electrical contact with the second rear face of the second sheet 70.

In some embodiments, the first electrode assembly 50a further includes a first layer of biocompatible conductive material 60 disposed on the first front face of the first sheet 70. Correspondingly, the second electrode assembly further includes a second layer of biocompatible conductive material 60 disposed on the second front face of the second sheet 70. As described above, the biocompatible conductive material 60 may be hydrogel or may be a conductive grease, conductive adhesive including the non-hydrogel conductive adhesives discussed above, conductive tape, conductive composite, etc.

In some embodiments, the first electrode assembly 50a further includes a first upper non-hydrogel conductive adhesive layer 80 (as described above) positioned between the first front face (skin-facing surface) of the first electrode element of the first electrode assembly 50a and the first rear face (outwardly facing surface) of the first sheet 70, such that the first electrode element of the first electrode assembly is in electrical contact with the outwardly facing surface of the sheet 70. Correspondingly, the second electrode assembly further includes a second upper non-hydrogel conductive adhesive layer 80 (as described above) positioned between the second front face of the second electrode element of the second electrode assembly and the second rear face of the second sheet 70, such that the second electrode element of the second electrode assembly is in electrical contact with the outwardly facing surface of sheet 70.

In some embodiments, each of the first and second sheets of anisotropic material 70 is a sheet of synthetic graphite. In some embodiments, each of the first and second sheets of anisotropic material 70 is a sheet of pyrolytic graphite. In other embodiments, each of the first and second sheets of anisotropic material 70 is graphite foil made from compressed high purity exfoliated mineral graphite or a sheet of graphitized polymer film. In other embodiments, the anisotropic material may be pyrolytic carbon. In other embodiments, the anisotropic material may be boron nitride. Other embodiments may utilize sheets of other conducting materials with anisotropic properties. In some embodiments (e.g., when the sheets of anisotropic material are sheets of synthetic graphite, such as pyrolytic graphite or compressed high purity exfoliated mineral graphite), the sheets of anisotropic material 70 are nonmetallic.

The alternating voltage between the first electrode assembly and the second electrode assembly may be applied by an AC voltage generator 820. In some embodiments, the frequency of the alternating voltage is between 50 kHz and 1 MHz, or between 100 kHz and 500 kHz. In the illustrated example, the AC voltage generator is controlled by a controller 822. The controller 822 may use temperature measurements to control the amplitude of the current to be delivered via the first and second electrode assemblies 50a in order to maintain temperatures below a safety threshold (e.g., 41°C). This may be accomplished, for example, by measuring a first temperature of the first electrode element, measuring a second temperature of the second electrode element, and controlling the applying of the alternating voltage based on the first temperature and the second temperature, as described below.

FIG. 7 depicts one example of hardware that is suitable for this purpose. More specifically, temperature sensors 800 (e.g., thermistors) are positioned in thermal contact with respective electrode elements (for example, dielectric material 310 / layer of metal 320) within each of the electrode assemblies 50a. The temperature sensors 800 measure respective first and second temperatures (e.g., at first and second electrode elements in the first electrode assembly and second electrode assembly, respectively), and the controller 822 controls the output of the AC voltage generator 820 based on these temperatures.

Similar embodiments and methods are envisaged utilizing any of the electrode assemblies 50a-e, or combinations thereof, in place of either or both of the first electrode assembly 50a and the second electrode assembly 50a.

### C. Upper Non-Hydrogel Conductive Adhesive Layer

For any embodiment, the upper non-hydrogel conductive adhesive layer (e.g., layer 80 in FIGs. 3, 6, and 7, and layers 380 in FIG. 5) can comprise any suitable conductive material that does not comprise a hydrogel, such that the electrode element(s) of the electrode assembly is/are in electrical contact with the outwardly facing surface of the layer of anisotropic material. In some embodiments, the non-hydrogel conductive adhesive layer does not comprise water.

In exemplary embodiments, the non-hydrogel conductive adhesive layer can comprise a dielectric material and conductive particles dispersed within the dielectric material. In some such embodiments, at least a portion of the conductive particles can define a conductive pathway through a thickness of the non-hydrogel conductive adhesive layer. For some embodiments, it is contemplated that the conductive particles can be aligned in response to application of an electric field such that the conductive particles undergo electrophoresis. In some embodiments, the dielectric material in the non-hydrogel conductive adhesive layer of each of the first and second electrode assemblies is a polymeric adhesive. Optionally, the polymeric adhesive can be an acrylic adhesive or a silicone adhesive.

In some embodiments, the conductive particles can comprise carbon in elemental (i.e. non-organic) form. Optionally, the conductive particles can comprise graphite powder. Additionally, or alternatively, the conductive particles can comprise carbon flakes. Additionally, or alternatively, the conductive particles can comprise carbon granules. Additionally, or alternatively, the conductive particles can comprise carbon fibers. Additionally, or alternatively, the conductive particles can comprise carbon nanotubes. Additionally, or alternatively, the conductive particles can comprise carbon black powder. Additionally, or alternatively, the conductive particles can comprise carbon nanowires. Additionally, or alternatively, the conductive particles can comprise carbon microcoils. In some embodiments, the conductive particles can comprise graphite powder, carbon black powder, carbon flakes, carbon granules, carbon fibers, carbon nanotubes, graphene nanotubes, single-walled carbon nanotubes, multi-walled carbon nanotubes, carbon nanowires, carbon microcoils, or any combination of these particles.

In exemplary embodiments, the conductive particles of the non-hydrogel conductive adhesive layer comprise a plurality of groups of conductive particles. In these embodiments, at least a portion of the conductive particles of some or all of the groups of conductive particles are aligned to define a conductive pathway through the thickness of the non-hydrogel conductive layer of each of the first and second electrode assemblies.

In some embodiments, the non-hydrogel conductive adhesive layer of an electrode assembly has a thickness ranging from about 20 µm to about 2,000 µm, such as from about 30 µm to about 1,000 µm, or from about 30 µm to about 70 µm. For example, the non-hydrogel conductive adhesive layer can have a thickness ranging from about 45 µm to about 55 µm.

In further embodiments, the non-hydrogel conductive adhesive layer further comprises a polar material (e.g., a polar salt). The polar salt may be a quaternary ammonium salt, such as a tetra alkyl ammonium salt. Exemplary non-hydrogel conductive adhesive materials, as well as methods for making such materials, are disclosed in U.S. Patent No. 8,673,184 and U.S. Patent No. 9,947,432. In exemplary embodiments, the non-hydrogel conductive adhesive material can be a dry carbon/salt adhesive, such as the OMNI-WAVE^{™} adhesive compositions manufactured and sold by FLEXCON (Spencer, MA, USA).

The non-hydrogel conductive adhesive layer may be a free-standing single layer of the conductive adhesive material, or, alternatively, may be present in the form of a tape, double-sided tape, or a scrim or mesh layer with the non-hydrogel conductive adhesive material on one or both sides of the scrim or mesh layer. In the latter case, the adhesive layers either side of the scrim or mesh may interdiffuse through the gaps in the scrim or mesh thereby forming continuous pathways through the scrim or mesh layer.

For all embodiments disclosed herein, the front (skin-contacting) layer of biocompatible conductive adhesive material may be a hydrogel or may be a non-hydrogel conductive adhesive as described above, and facilitates electrical contact of the layer of anisotropic material with the skin.

In further aspects, by avoiding the use of hydrogel within the upper conductive layer, and particularly by avoiding the use of hydrogel within both the upper conductive layer and the front (skin-contacting) layer of biocompatible conductive adhesive material, it is contemplated that electrode assemblies comprising conductive adhesive materials do not require moisture barrier packaging, making the cost of packaging far more affordable. Additionally, it is contemplated that the conductive adhesive materials of the disclosed electrode assemblies can avoid the signal variation issues of hydrogels, thereby providing consistent material properties (e.g., tackiness) and reliable performance during delivery of TTFields. Further, it is contemplated that the disclosed conductive adhesive composites can have a far greater shelf life than hydrogel-containing materials, decreasing the frequency at which electrode assemblies (or the skin contact layers of electrode assemblies) must be replaced.

Features and advantages of this disclosure are apparent from the detailed specification, and the claims cover all such features and advantages. Numerous variations will occur to those skilled in the art, and any variations equivalent to those described in this disclosure fall within the scope of this disclosure. Those skilled in the art will appreciate that the conception upon which this disclosure is based may be used as a basis for designing other methods and systems for carrying out the several purposes of this disclosure. As a result, the claims should not be considered as limited by the description or examples.

## Claims

1. An apparatus, comprising:
at least one electrode element (E1, E2) having a skin-facing surface;
a layer of anisotropic material (70) having a skin-facing surface and an opposing outwardly-facing surface;
a first layer (80; 380) of conductive adhesive not including a crosslinked hydrophilic polymer positioned between the skin-facing surface of the at least one electrode element (E1, E2) and the outwardly-facing surface of the layer of anisotropic material (70);
a skin-contact layer (60) comprising a biocompatible conductive adhesive, wherein the skin-contact layer (60) is disposed on a skin-facing side of the layer of anisotropic material (70);
wherein the first layer (80; 380) of conductive adhesive facilitates electrical contact between the skin-facing surface of the at least one electrode element (E1, E2) and the outwardly-facing surface of the layer of anisotropic material (70).

2. The apparatus of claim 1, wherein the conductive adhesive of the skin-contact layer (60) comprises a hydrogel.

3. The apparatus of claim 1, wherein the conductive adhesive of the skin-contact layer (60) is a conductive adhesive not including a crosslinked hydrophilic polymer.

4. The apparatus of claim 3, wherein the conductive adhesive of the skin-contact layer (60) differs from the conductive adhesive of the first layer (80; 380) of conductive adhesive.

5. The apparatus of claim 1, wherein the first layer (80; 380) of conductive adhesive comprises a material that facilitates electrical conductivity in a z-direction that is perpendicular to the plane of the layer of anisotropic material (70).

6. The apparatus of claim 1, wherein the first layer (80; 380) of conductive adhesive comprises a dielectric material and conductive particles dispersed within the dielectric material.

7. The apparatus of claim 6, wherein the conductive particles comprise carbon in elemental, non-organic, form.

8. The apparatus of claim 6, wherein the conductive particles comprise carbon flakes, carbon granules, carbon nanotubes, single-walled carbon nanotubes, multi-walled carbon nanotubes, carbon black powder, graphite powder, carbon nanowires, carbon microcoils or any combination thereof.

9. The apparatus of claim 5, wherein the material that facilitates electrical conductivity in the z-direction is a conductive material having 3D carbon structures.

10. The apparatus of claim 9, wherein the material that facilitates electrical conductivity in the z-direction is carbon microcoils.

11. The apparatus of claim 5, wherein the material that facilitates electrical conductivity in the z-direction is a capacitive material.

12. The apparatus of claim 3, wherein either, or both, of the first layer (80; 380) of conductive adhesive or the conductive adhesive of the skin-contact layer (60) further comprises a polar material.

13. The apparatus of claim 3, wherein either, or both, of the first layer (80; 380) of conductive adhesive or the skin-contact layer (60) further comprises a scrim or mesh layer, with the conductive adhesive on one or both sides of the scrim or mesh layer.

14. The apparatus of claim 1, wherein the layer of anisotropic material (70) has one or both of a first thermal conductivity in a direction that is perpendicular to the plane of the layer of anisotropic material (70) and a thermal conductivity in directions that are parallel to the plane of the layer of anisotropic material (70) is more than two times higher than the first thermal conductivity, or a first resistance in a direction that is perpendicular to the plane of the layer of anisotropic material (70) and a resistance in directions that are parallel to the plane of the layer of anisotropic material (70) is less than half the first resistance.

15. The apparatus of claim 6, wherein the dielectric material is a polymeric adhesive.

16. The apparatus of claim 1, wherein the skin-contact layer (60) is disposed on the skin-facing surface of the layer of anisotropic material (70).

## Patentansprüche

1. Einen Apparat, der Folgendes umfasst:
mindestens ein Elektrodenelement (E1, E2) mit einer der Haut zugewandten Oberfläche;
eine Schicht aus anisotropem Material (70) mit einer der Haut zugewandten Oberfläche und einer gegenüberliegenden, nach außen gerichteter Oberfläche;
eine erste Schicht (80; 380) aus leitfähigem Klebstoff, der kein vernetztes hydrophiles Polymer enthält, die zwischen welcher der Haut zugewandten Oberfläche des mindestens einen Elektrodenelements (E1, E2) und der nach außen gerichteter Oberfläche der Schicht aus anisotropem Material (70) angeordnet ist;
eine Hautkontaktschicht (60), die einen biokompatiblen leitfähigen Klebstoff umfasst, wobei die Hautkontaktschicht (60) auf einer der Haut zugewandten Seite der Schicht aus anisotropem Material (70) angeordnet ist;
wobei die erste Schicht (80; 380) aus leitfähigem Klebstoff den elektrischen Kontakt zwischen der Haut zugewandten Oberfläche des mindestens einen Elektrodenelements (E1, E2) und der nach außen gerichteter Oberfläche der Schicht aus anisotropem Material (70) erleichtert.

2. Der Apparat gemäß Anspruch 1, wobei der leitfähige Klebstoff der Hautkontaktschicht (60) ein Hydrogel umfasst.

3. Der Apparat gemäß Anspruch 1, wobei der leitfähige Klebstoff der Hautkontaktschicht (60) ein leitfähiger Klebstoff ist, der kein vernetztes hydrophiles Polymer enthält.

4. Der Apparat gemäß Anspruch 3, wobei sich der leitfähige Klebstoff der Hautkontaktschicht (60) von dem leitfähigen Klebstoff der ersten Schicht (80; 380) aus leitfähigem Klebstoff unterscheidet.

5. Der Apparat gemäß Anspruch 1, wobei die erste Schicht (80; 380) aus leitfähigem Klebstoff ein Material umfasst, das die elektrische Leitfähigkeit in einer z-Richtung erleichtert, die senkrecht zur Ebene der Schicht aus anisotropem Material (70) verläuft.

6. Der Apparat gemäß Anspruch 1, wobei die erste Schicht (80; 380) aus leitfähigem Klebstoff ein dielektrisches Material und in dem dielektrischen Material dispergierte leitfähige Partikel umfasst.

7. Der Apparat gemäß Anspruch 6, wobei die leitfähigen Teilchen Kohlenstoff in elementarer, nicht-organischer Form umfassen.

8. Der Apparat gemäß Anspruch 6, wobei die leitfähigen Partikel Kohlenstoffflocken, Kohlenstoffgranulat, Kohlenstoffnanoröhren, einwandige Kohlenstoffnanoröhren, mehrwandige Kohlenstoffnanoröhren, Rußpulver, Graphitpulver, Kohlenstoffnanodrähte, Kohlenstoffmikrospulen oder eine beliebige Kombination davon umfassen.

9. Der Apparat gemäß Anspruch 5, wobei das Material, das die elektrische Leitfähigkeit in der Z-Richtung erleichtert, ein leitfähiges Material mit 3D-Kohlenstoffstrukturen ist.

10. Der Apparat gemäß Anspruch 9, wobei das Material, das die elektrische Leitfähigkeit in der Z-Richtung erleichtert, aus Kohlenstoff-Mikrospulen besteht.

11. Der Apparat gemäß Anspruch 5, wobei das Material, das die elektrische Leitfähigkeit in der Z-Richtung erleichtert, ein kapazitives Material ist.

12. Der Apparat gemäß Anspruch 3, wobei entweder die erste Schicht (80; 380) aus leitfähigem Klebstoff oder der leitfähige Klebstoff der Hautkontaktschicht (60) oder beide ein polares Material enthalten.

13. Der Apparat gemäß Anspruch 3, bei der entweder die erste Schicht (80; 380) aus leitfähigem Klebstoff oder die Hautkontaktschicht (60) oder beide eine Gitter- oder Netzschicht umfassen, wobei sich der leitfähige Klebstoff auf einer oder beiden Seiten der Gitter- oder Netzschicht befindet.

14. Der Apparat gemäß Anspruch 1, wobei die Schicht aus anisotropem Material (70) eine oder beide der folgenden Eigenschaften aufweist: eine erste Wärmeleitfähigkeit in einer Richtung, die senkrecht zur Ebene der Schicht aus anisotropem Material (70) liegt, und eine Wärmeleitfähigkeit in Richtungen, die parallel zur Ebene der Schicht aus anisotropem Material (70) liegen, die mehr als doppelt so hoch ist wie die erste Wärmeleitfähigkeit, oder ein erster Widerstand in einer Richtung, die senkrecht zur Ebene der Schicht aus anisotropem Material (70) ist, und ein Widerstand in Richtungen, die parallel zur Ebene der Schicht aus anisotropem Material (70) sind, weniger als die Hälfte des ersten Widerstands beträgt.

15. Der Apparat gemäß Anspruch 6, wobei das dielektrische Material ein polymerer Klebstoff ist.

16. Der Apparat gemäß Anspruch 1, wobei die Hautkontaktschicht (60) auf der zur Haut zugewandten Oberfläche der Schicht aus anisotropem Material (70) angeordnet ist.

## Revendications

1. Appareil, comprenant :
au moins un élément d'électrode (E1, E2) présentant une surface tournée vers la peau ;
une couche de matériau anisotrope (70) présentant une surface tournée vers la peau et une surface opposée tournée vers l'extérieur ;
une première couche (80 ; 380) d'adhésif conducteur n'incluant pas de polymère hydrophile réticulé positionnée entre la surface tournée vers la peau de l'au moins un élément d'électrode (E1, E2) et la surface tournée vers l'extérieur de la couche de matériau anisotrope (70) ;
une couche de contact avec la peau (60) comprenant un adhésif conducteur biocompatible, où la couche de contact avec la peau (60) est disposée sur un côté tourné vers la peau de la couche de matériau anisotrope (70) ;
dans lequel la première couche (80 ; 380) d'adhésif conducteur facilite le contact électrique entre la surface tournée vers la peau de l'au moins un élément d'électrode (E1, E2) et la surface tournée vers l'extérieur de la couche de matériau anisotrope (70).

2. Appareil selon la revendication 1, dans lequel l'adhésif conducteur de la couche de contact avec la peau (60) comprend un hydrogel.

3. Appareil selon la revendication 1, dans lequel l'adhésif conducteur de la couche de contact avec la peau (60) est un adhésif conducteur n'incluant pas de polymère hydrophile réticulé.

4. Appareil selon la revendication 3, dans lequel l'adhésif conducteur de la couche de contact avec la peau (60) diffère de l'adhésif conducteur de la première couche (80 ; 380) d'adhésif conducteur.

5. Appareil selon la revendication 1, dans lequel la première couche (80 ; 380) d'adhésif conducteur comprend un matériau qui facilite la conductivité électrique dans une direction z qui est perpendiculaire au plan de la couche de matériau anisotrope (70).

6. Appareil selon la revendication 1, dans lequel la première couche (80 ; 380) d'adhésif conducteur comprend un matériau diélectrique et des particules conductrices dispersées dans le matériau diélectrique.

7. Appareil selon la revendication 6, dans lequel les particules conductrices comprennent du carbone sous forme élémentaire non organique.

8. Appareil selon la revendication 6, dans lequel les particules conductrices comprennent des flocons de carbone, des granulés de carbone, des nanotubes de carbone, des nanotubes de carbone à paroi unique, des nanotubes de carbone à parois multiples, de la poudre de noir de carbone, de la poudre de graphite, des nanofils de carbone, des microbobines de carbone ou toute combinaison de ceux-ci.

9. Appareil selon la revendication 5, dans lequel le matériau qui facilite la conductivité électrique dans la direction z est un matériau conducteur présentant des structures de carbone 3D.

10. Appareil selon la revendication 9, dans lequel le matériau qui facilite la conductivité électrique dans la direction z est constitué de microbobines de carbone.

11. Appareil selon la revendication 5, dans lequel le matériau qui facilite la conductivité électrique dans la direction z est un matériau capacitif.

12. Appareil selon la revendication 3, dans lequel l'un ou les deux parmi la première couche (80 ; 380) d'adhésif conducteur ou l'adhésif conducteur de la couche de contact avec la peau (60) comprend/comprennent en outre un matériau polaire.

13. Appareil selon la revendication 3, dans lequel l'une ou les deux parmi la première couche (80 ; 380) d'adhésif conducteur ou la couche de contact avec la peau (60) comprend/comprennent en outre une couche de canevas ou de maille, l'adhésif conducteur étant sur l'un ou les deux côtés de la couche de canevas ou de maille.

14. Appareil selon la revendication 1, dans lequel la couche de matériau anisotrope (70) présente l'une ou les deux parmi une première conductivité thermique dans une direction qui est perpendiculaire au plan de la couche de matériau anisotrope (70) et une conductivité thermique dans des directions qui sont parallèles au plan de la couche de matériau anisotrope (70) est plus de deux fois supérieure à la première conductivité thermique, ou une première résistance dans une direction qui est perpendiculaire au plan de la couche de matériau anisotrope (70) et une résistance dans des directions qui sont parallèles au plan de la couche de matériau anisotrope (70) est inférieure à la moitié de la première résistance.

15. Appareil selon la revendication 6, dans lequel le matériau diélectrique est un adhésif polymère.

16. Appareil selon la revendication 1, dans lequel la couche de contact avec la peau (60) est disposée sur la surface tournée vers la peau de la couche de matériau anisotrope (70).
